(19) **Europäisches Patentamt / European Patent Office / Office européen des brevets**

(11) **EP 4 501 447 A1**

(12) **EUROPEAN PATENT APPLICATION**
published in accordance with Art. 153(4) EPC

(43) Date of publication:
05.02.2025 Bulletin 2025/06

(21) Application number: 23780500.7

(22) Date of filing: 28.03.2023

(51) International Patent Classification (IPC):
$B01J\ 20/26^{(2006.01)}$   $A61F\ 13/15^{(2006.01)}$
$A61F\ 13/505^{(2006.01)}$   $A61F\ 13/53^{(2006.01)}$
$B01J\ 20/30^{(2006.01)}$

(52) Cooperative Patent Classification (CPC):
A61F 13/15; A61F 13/505; A61F 13/53;
B01J 20/26; B01J 20/30

(86) International application number:
PCT/JP2023/012459

(87) International publication number:
WO 2023/190491 (05.10.2023 Gazette 2023/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.03.2022 JP 2022057397

(71) Applicant: Sumitomo Seika Chemicals Co., Ltd.
Kako-gun, Hyogo 675-0145 (JP)

(72) Inventors:
• AWAJI, Naoya
Himeji-shi, Hyogo 672-8076 (JP)
• GOGA, Yuki
Himeji-shi, Hyogo 672-8076 (JP)

(74) Representative: Mewburn Ellis LLP
Aurora Building
Counterslip
Bristol BS1 6BX (GB)

(54) **WATER-ABSORBING RESIN COMPOSITION, PRODUCTION METHOD THEREFOR, ABSORBER, AND ABSORBENT ARTICLE**

(57) Provided is a water-absorbing resin composition having both gel stability of the water-absorbing resin composition when a hygienic material is usually worn and high handleability when the water-absorbing resin composition is subjected to a separation treatment after being irradiated with ultraviolet rays as a waste treatment means. The water-absorbing resin composition including water-absorbent resin particles and a chelating agent. The water-absorbent resin particles are surface-crosslinked, and the chelating agent has an iron (III) chelate stability constant of 16 logKML or more and 24 logKML or less, and 4 or more and 10 or less carboxyl groups.

EP 4 501 447 A1

## Description

Technical Field

The present invention relates to a water-absorbing resin composition, a method for producing the same, an absorber, and an absorbent article, and more particularly to a water-absorbing resin composition constituting an absorber suitably used for hygienic materials such as disposable diapers, sanitary napkins, and incontinence pads, a method for producing the water-absorbing resin composition, an absorber, and an absorbent article.

Background Art

In recent years, water-absorbing resins have been widely used in the field of hygienic materials such as disposable diapers, sanitary napkins, and incontinence pads.

As such a water-absorbing resin, a partially neutralized acrylic acid polymer crosslinked product has an excellent water absorption capacity, and acrylic acid as a raw material thereof is easily industrially available, so that the water-absorbing resin can be produced at a low cost with a constant quality, and is considered to be a preferable water-absorbing resin because it has many advantages such as resistance to decay and deterioration.

Citation List

Patent Literature

PTL 1: Japanese Patent Laid-open Publication No. 2005-29751

Summary of Invention

Technical Problem

A gel formed by allowing a water-absorbing resin to absorb a body fluid such as human urine generally deteriorates over time, so that the absorbability of the gel decreases. When the deterioration of the gel further progresses, the water-absorbing resin itself is partially decomposed, so that the water-soluble dissolved matter is eluted from the gel. When a product including the water-absorbing resin is used in contact with a human body, a water-soluble dissolved matter exuded from the product may adhere to the skin, leading to the occurrence of a rash or the like (see PTL 1). Therefore, in the hygienic material, the water-absorbing resin is required to have gel stability during normal wearing.

An absorbent article such as a disposable diaper, a sanitary napkin, or an incontinence pad mainly includes an absorber that absorbs and holds body fluids such as urine and menstrual blood excreted from the body, which is disposed in a central portion, a liquid-permeable surface sheet (top sheet) disposed on a side in contact with the body, and a liquid-impermeable underside sheet (back sheet) disposed on an opposite side in contact with the body. The absorber is usually composed of hydrophilic fibers such as pulp and a water-absorbing resin.

In a method for separating the water-absorbing resin from the absorber when discarding the absorbent article after use, it is conceivable that the gel strength of the water-absorbing resin is moderately reduced by some means to facilitate the separation of the gel from other materials (hydrophilic fibers and the like) contained in the absorber, thereby improving the handleability. As means therefor, ultraviolet irradiation, and the addition of a gel degradation accelerator, and the like are used.

However, when the gel stability of the water-absorbing resin is enhanced, depending on the degree of the gel stability, there is a problem that the gel strength is less likely to decrease even when means such as ultraviolet irradiation is used.

Under such circumstances, a main object of the present invention is to provide a water-absorbing resin composition having both gel stability of the water-absorbing resin composition when a hygienic material is usually worn and high handleability when the water-absorbing resin composition is subjected to a separation treatment after being irradiated with ultraviolet rays as a waste treatment means.

Solution to Problem

The present inventors have conducted intensive studies in order to solve the above problems. As a result, the present inventors have found that the above problems can be solved by using a water-absorbing resin composition containing surface-crosslinked water-absorbent resin particles and a chelating agent, in which the chelating agent satisfies a predetermined iron (III) chelate stability constant and number of carboxyl groups. The present invention was accomplished by further conducting extensive research, based on this finding.

That is, the present invention provides aspects of the invention having the following configurations. Item 1. A water-absorbing resin composition including water-absorbent resin particles and a chelating agent, in which the water-absorbent resin particles are surface-crosslinked, and the chelating agent has an iron (III) chelate stability constant of 16 logKML or more and 24 logKML or less, and 4 or more and 10 or less carboxyl groups. Item 2. The water-absorbing resin composition according to item 1, having a gel strength change degree after UV irradiation of 40% or more.

(Gel strength change degree after UV irradiation)

According to procedures 1 and 2 below, the gel strength change degree (%) after UV irradiation is calculated by a formula below from an initial value of obtained gel strength (gel strength (A)) and a value of the gel strength after ultraviolet irradiation (gel strength (C)).

gel strength change degree (%) after UV irradiation = [(gel strength (A) - gel strength (C))/gel strength (A)] $\times$ 100

(Initial value of gel strength (gel strength (A))

A swollen gel prepared by the following procedure 1 is allowed to stand under an environment of a temperature of $25\pm2°C$ and a relative humidity of $50\pm10\%$ for 60 minutes after preparation, and then the gel strength (initial value of gel strength) is measured by the following procedure 2. The value of the gel strength at this time is defined as gel strength (A).

(Value of gel strength after ultraviolet irradiation (gel strength (C))

The swollen gel prepared by the following procedure 1 is allowed to stand under an environment of a temperature of $37\pm2°C$ and a relative humidity of $60\pm10\%$ for 14 hours after preparation. Thereafter, the swollen gel after being allowed to stand is placed directly under a light source of an ultraviolet irradiation device under an environment of a temperature of $25\pm2°C$ and a relative humidity of $50\pm10\%$. At this time, a distance between a surface of the swollen gel and the light source is set to 7 cm. After covering the entire device with a box to shield light, the swollen gel in a beaker is irradiated with ultraviolet rays having a wavelength of 254 nm for 150 minutes. After the ultraviolet irradiation, the gel strength is measured by the following procedure 2.

A value of the gel strength at this time is defined as gel strength (C). Procedure 1 (preparation of swollen gel)

In a beaker having an internal volume of 100 mL, 39.0 g of artificial urine is weighed, magnetic stirrer bars are put in the beaker, and the beaker is placed on a magnetic stirrer to rotate the magnetic stirrer bars at 600 rpm. Next, 1.00 g of the water-absorbing resin composition is put into the beaker under stirring, and the stirring is performed until a rotating vortex disappears and a liquid level becomes horizontal, thereby preparing a swollen gel as a measurement sample. Immediately after preparing the swollen gel, the beaker containing the swollen gel is covered with a wrap.

Procedure 2 (measurement of gel strength)

A gel strength value ($N/m^2$) is measured using a card meter under conditions of a temperature and standing time of the initial value of the gel strength (gel strength (A)) and the value of the gel strength after ultraviolet irradiation (gel strength (C)).

Item 3. The water-absorbing resin composition according to item 1 or 2, in which a content of the chelating agent is 0.001 parts by mass or more and 2 parts by mass or less with respect to 100 parts by mass of the water-absorbent resin particles.
Item 4. The water-absorbing resin composition according to any one of items 1 to 3, in which the water-absorbing resin composition has a physiological saline retention amount of 25 g/g or more and 60 g/g or less.
Item 5. The water-absorbing resin composition according to any one of items 1 to 4, in which the water-absorbing resin composition has a physiological saline absorption amount of 5 ml/g or more and 40 ml/g or less under a load of 4.14 kPa.
Item 6. An absorber including the water-absorbing resin composition according to any one of items 1 to 5.
Item 7. An absorbent article including the absorber according to item 6.
Item 8. A method for producing the water-absorbing resin composition according to any one of items 1 to 5, the method including: mixing the water-absorbent resin particles before surface-crosslinking with the chelating agent; and/or mixing the water-absorbent resin particles after surface-crosslinking with the chelating agent.

Advantageous Effects of Invention

The present invention can provide a water-absorbing resin composition having both gel stability of the water-absorbing resin composition when a hygienic material is usually worn and high handleability when the water-absorbing resin composition is subjected to a separation treatment after being irradiated with ultraviolet rays as a waste treatment means. Furthermore, the present invention can also provide a method for producing the water-absorbing resin composition, an absorber using the water-absorbing resin composition, and an absorbent article.

Brief Description of Drawings

[Fig. 1] Fig. 1 is a schematic view of a device for measuring a physiological saline absorption amount under a load of 4.14 kPa.
[Fig. 2] Fig. 2 is a schematic view of a measuring device used for measuring gel strength.

Description of Embodiments

In the present description, the term "comprising" includes "consisting essentially of" and "consisting of". In the present description, the term "(meth)acryl" means "acryl or methacryl", the term "(meth)acrylate" means "acrylate or methacrylate". The term "water-soluble" means that a solubility of 5 mass% or more is exhibited in water at 25°C.
In the present description, two numerical values joined by "to" means a numerical range including the numerical values before and after "to" as a lower limit value and an upper limit value, respectively. When a plurality of lower limit values and a plurality of upper limit values are described separately, an arbitrary lower limit value and an arbitrary upper limit value may be selected and joined by "to".

1. Water-Absorbing Resin Composition

A water-absorbing resin composition according to the present invention includes water-absorbent resin particles and a chelating agent, in which the water-absorbent resin particles are surface-crosslinked, and the chelating agent has an iron (III) chelate stability constant of 16 logKML or more and 24 logKML or less, and 4 or more and 10 or less carboxyl groups (hereinafter, the chelating agent satisfying this property may be referred to as "chelating agent A"). The water-absorbing resin composition according to the present invention having such characteristics has both gel stability of the water-absorbing resin composition when a hygienic material is usually worn and high handleability when the water-absorbing resin composition is subjected to a separation treatment after being irradiated with ultraviolet rays as a waste treatment means. Hereinafter, the water-absorbing resin composition according to the present invention will be described in detail.

(Chelating Agent)

The chelating agent A contained in the water-absorbing resin composition according to the present invention has an iron (III) chelate stability constant of 16 logKML or more and 24 logKML or less, and 4 or more and 10 or less carboxyl groups. Literature values can be used as the iron (III) chelate stability constant of the chelating agent.
From the viewpoint of more suitably exhibiting the effect of the present invention, the iron (III) chelate stability constant of the chelating agent A is preferably 16 logKML or more, more preferably 18 logKML or more, still more preferably 19 logKML or more, and yet still more preferably 19.5 logKML or more, and is preferably 24 logKML or less, more preferably 22 logKML or less, more preferably 21 logKML or less, and still more preferably 20.4 logKML or less. The range of the iron (III) chelate stability constant is preferably 16 to 24 logKML, 19 to 21 logKML, and 19.5 to 20.4 logKML and the like.
The chelating agent A contained in the water-absorbing resin composition according to the present invention has 4 or more and 10 or less carboxyl groups. From the viewpoint of more suitably exhibiting the effect of the present invention, the number of carboxyl groups of the chelating agent is preferably 4 to 10, more preferably 4 to 6, still more preferably 4 to 5, and yet still more preferably 4.
Preferable specific examples of the chelating agent A contained in the water-absorbing resin composition according to the present invention include ethylenediamine-N,N'-disuccinic acid and glycol ether diamine tetraacetic acid. The chelating agent A may be present in the form of a salt. Examples of the salt include a sodium salt and a potassium salt. The chelating agent contained in the water-absorbing resin composition according to the present invention may be one or two or more. The water-absorbing resin composition according to the present invention may or may not contain other chelating agent different from the chelating agent A (that is, a chelating agent which does not satisfy the property that the iron (III) chelate stability constant is 16 logKML or more and 24 logKML or less and the number of carboxyl groups is 4 or more and 10 or less). As the other chelating agent, a known chelating agent added to the water-absorbing resin can be used. From the viewpoint of more suitably exhibiting the effect of the present invention, the proportion of the chelating agent A in the chelating agent contained in the water-absorbing resin composition according to the present invention is preferably 60 mass% or more, more preferably 70 mass% or more, still more preferably 80 mass% or more, yet still more preferably 90 mass% or more, further preferably 95 mass% or more, and further more preferably 100 mass% (that is, the chelating agent contained in the water-absorbing resin composition according to the present invention is only the chelating agent A).

In the water-absorbing resin composition according to the present invention, the content of the chelating agent A is preferably 0.001 parts by mass or more, and more preferably 0.01 parts by mass or more, and preferably 2 parts by mass or less, and more preferably 1 part by mass or less, with respect to 100 parts by mass of the water-absorbent resin particles. The range of the content of the chelating agent A is preferably 0.001 to 2 parts by mass and 0.01 to 1 part by mass and the like.

The chelating agent A is preferably present on at least one of the surface and inside of the surface-crosslinked water-absorbent resin particles, and more preferably present on at least the surface. For example, by mixing the surface-crosslinked water-absorbent resin particles and the chelating agent A in a solid phase state, the chelating agent A can be present on the surface of the surface-crosslinked water-absorbent resin particles to such an extent that the effect of the present invention can be exhibited. The water-absorbing resin composition of the present invention may be prepared by mixing the chelating agent A with the surface-crosslinked water-absorbent resin particles in a state of being dissolved or dispersed in a liquid medium such as an aqueous liquid. The chelating agent A may be contained inside the surface-crosslinked water-absorbent resin particles.

Next, the water-absorbent resin particles contained in the water-absorbing resin composition according to the present invention will be described in detail.

(Water-Absorbent Resin Particles)

The water-absorbent resin particles contained in the water-absorbing resin composition according to the present invention are formed by crosslinking a polymer of a water-soluble ethylenically unsaturated monomer, that is, a crosslinked polymer having a structural unit derived from a water-soluble ethylenically unsaturated monomer. The water-absorbent resin particles are surface-crosslinked. As the surface treatment method, for example, a method described in <Surface Crosslinking Step> to be described later can be adopted.

From the viewpoint of more suitably exhibiting the effect of the present invention, the physiological saline retention amount of the water-absorbent resin particles is preferably 20 g/g or more, more preferably 25 g/g or more, and still more preferably 30 g/g or more, and is preferably 60 g/g or less, more preferably 55 g/g or less, and still more preferably 50 g/g or less. The range of the physiological saline retention amount is preferably 25 to 60 g/g and 30 to 50 g/g and the like.

From the viewpoint of more suitably exhibiting the effect of the present invention, the physiological saline absorption amount of the water-absorbent resin particles under a load of 4.14 kPa is preferably 5 ml/g or more, more preferably 10 ml/g or more, and still more preferably 15 ml/g or more, and is preferably 40 ml/g or less, more preferably 35 ml/g or less, and still more preferably 30 ml/g or less. The range of the physiological saline absorption amount is preferably 5 to 40 ml/g and 10 to 30 ml/g and the like.

Each of the physiological saline retention amount and the physiological saline absorption amount under a load of 4.14 kPa of the water-absorbent resin particles is a value measured by the method described in Examples.

The water-absorbent resin is usually in a particle form. From the viewpoint of suitably exhibiting the effect of the present invention while avoiding local absorption in the absorbent article, the median particle diameter of the water-absorbent resin particles is preferably 150 μm or more, 200 μm or more, 250 μm or more, 280 μm or more, 300 μm or more, or 350 μm or more. From the viewpoint of suitably exhibiting the effect of the present invention while making the tactile sensation of the absorbent article comfortable, the median particle diameter is preferably 850 μm or less, 600 μm or less, 550 μm or less, 500 μm or less, 450 μm or less, or 400 μm or less. That is, the median particle diameter is preferably 150 to 850 μm, more preferably 200 to 600 μm, still more preferably 250 to 500 μm, yet still more preferably 300 to 450 μm, and further preferably 320 to 400 μm. Also in the water-absorbing resin composition according to the present invention, the median particle diameter is preferably 150 to 850 μm, more preferably 200 to 600 μm, still more preferably 250 to 500 μm, yet still more preferably 300 to 450 μm, and further preferably 320 to 400 μm.

The water-absorbent resin particles may be in a form in which fine particles (primary particles) are aggregated (secondary particles) in addition to a form in which water-absorbent resin particles are single particles. Examples of the shape of the primary particles include a substantially spherical shape, an indefinite crushed shape, and a plate shape. Examples of the primary particles produced by reversed phase suspension polymerization include substantially spherical single particles having a smooth surface shape such as a perfect spherical shape or an elliptical spherical shape.

The median particle diameter of the water-absorbent resin particles can be measured using a JIS standard sieve.

Specifically, the median particle diameter is a value measured by the method described in Examples.

As a polymerization method of the water-soluble ethylenically unsaturated monomer, an aqueous solution polymerization method, an emulsion polymerization method, and a reversed phase suspension polymerization method and the like, which are representative polymerization methods, are used. In the aqueous solution polymerization method, polymerization is performed by heating a water-soluble ethylenically unsaturated monomer aqueous solution while stirring the aqueous solution as necessary. In the reversed phase suspension polymerization method, polymerization is performed by heating a water-soluble ethylenically unsaturated monomer in a hydrocarbon dispersion medium under stirring.

An example of a method for producing the water-absorbent resin particles will be described below.

Specific examples of the method for producing water-absorbent resin particles include a method for producing water-absorbent resin particles by subjecting a water-soluble ethylenically unsaturated monomer to reversed-phase suspension polymerization in a hydrocarbon dispersion medium, the method including the steps of: performing polymerization in the presence of a radical polymerization initiator; and surface-crosslinking a hydrous gel obtained by the polymerization in the presence of a surface crosslinking agent. In the method for producing water-absorbent resin particles according to the present invention, an internal crosslinking agent may be added to the water-soluble ethylenically unsaturated monomer as necessary to form a hydrous gel having an internal crosslinked structure.

<Polymerization Step>

[Water-Soluble Ethylenically Unsaturated Monomer]

Examples of the water-soluble ethylenically unsaturated monomer include (meth)acrylic acid (in the present specification, "acrylic" and "methacrylic" are collectively referred to as "(meth)acrylic". The same applies hereinafter) and salts thereof; 2-(meth)acrylamido-2-methylpropane sulfonic acid and salts thereof; nonionic monomers such as (meth) acrylamide, N,N-dimethyl(meth)acrylamide, 2-hydroxyethyl (meth)acrylate, N-methylol(meth)acrylamide, and polyethylene glycol mono(meth)acrylate; and amino group-containing unsaturated monomers such as N,N-diethylaminoethyl (meth)acrylate, N,N-diethylaminopropyl (meth)acrylate, and diethylaminopropyl(meth)acrylamide and quaternized products thereof. Among these water-soluble ethylenically unsaturated monomers, (meth)acrylic acid or a salt thereof, (meth) acrylamide, and N,N-dimethylacrylamide are preferable, and (meth)acrylic acid and a salt thereof are more preferable from the viewpoint of industrial availability and the like. These water-soluble ethylenically unsaturated monomers may be used singly or in combination of two or more kinds thereof.

Among them, acrylic acid and salts thereof are widely used as raw materials of water-absorbent resin particles, and these acrylic acid and/or salts thereof may be copolymerized with another one of the above water-soluble ethylenically unsaturated monomers. In this case, acrylic acid and/or a salt thereof is preferably used as a main water-soluble ethylenically unsaturated monomer in an amount of 70 to 100 mol% with respect to the total amount of water-soluble ethylenically unsaturated monomers.

The water-soluble ethylenically unsaturated monomer may be dispersed in a hydrocarbon dispersion medium in a state of an aqueous solution, and subjected to reversed phase suspension polymerization. When the water-soluble ethylenically unsaturated monomer is in an aqueous solution, the dispersion efficiency in the hydrocarbon dispersion medium can be increased. The concentration of the water-soluble ethylenically unsaturated monomer in this aqueous solution is preferably in the range of 20 mass% to a saturated concentration. The concentration of the water-soluble ethylenically unsaturated monomer is more preferably 55 mass% or less, still more preferably 50 mass% or less, and yet still more preferably 45 mass% or less. Meanwhile, the concentration of the water-soluble ethylenically unsaturated monomer is more preferably 25 mass% or more, still more preferably 30 mass% or more, and yet still more preferably 35 mass% or more.

When the water-soluble ethylenically unsaturated monomer has an acid group such as (meth)acrylic acid or 2-(meth)acrylamide-2-methylpropanesulfonic acid, a compound in which the acid group has been neutralized in advance with an alkaline neutralizing agent as necessary may be used. Examples of such an alkaline neutralizing agent include alkali metal salts such as sodium hydroxide, sodium carbonate, sodium hydrogen carbonate, potassium hydroxide, and potassium carbonate; and ammonia. These alkaline neutralizing agents may be used in the form of an aqueous solution in order to simplify the neutralization operation. The above alkaline neutralizing agents may be used singly or in combination of two or more kinds thereof.

The neutralization degree of the water-soluble ethylenically unsaturated monomer with the alkaline neutralizing agent is preferably 10 to 100 mol%, more preferably 30 to 90 mol%, still more preferably 50 to 85 mol%, and yet still more preferably 70 to 80 mol% as the neutralization degree with respect to all the acid groups of the water-soluble ethylenically unsaturated monomer.

[Radical Polymerization Initiator]

Examples of the radical polymerization initiator added in the polymerization step include persulfates such as potassium persulfate, ammonium persulfate, and sodium persulfate, peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide, 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, and 2,2'-azobis{2-[1-(2-hydroxyethyl)-2-imidazolin-2-yl]propane}dihydrochloride, and azo compounds such as 2,2"-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Among these radical polymerization initiators, potassium persulfate, ammonium persulfate, sodium persulfate, and 2,2'-azobis(2-amidinopropane)dihydrochloride are preferable from the viewpoint of easy availability and easy handling. These radical polymerization initiators may be used singly or in combination of two or more. The radical polymerization initiator can also be used as a redox polymerization initiator in combination with a reducing agent such as sodium sulfite, sodium hydrogen sulfite, ferrous sulfate, or L-ascorbic acid.

The amount of the radical polymerization initiator used is, for example, 0.00005 to 0.01 mol with respect to 1 mol of the water-soluble ethylenically unsaturated monomer. By satisfying such a use amount, occurrence of a rapid polymerization reaction can be avoided, and the polymerization reaction can be completed in an appropriate time.

[Internal Crosslinking Agent]

Examples of the internal crosslinking agent include those capable of crosslinking a polymer of a water-soluble ethylenically unsaturated monomer to be used such as unsaturated polyesters obtained by reacting polyols such as diols and triols such as (poly)ethylene glycol ["(poly)" means with or without a "poly" prefix. The same applies hereinafter], (poly)propylene glycol, 1,4-butanediol, 1,6-hexanediol, trimethylolpropane, and (poly)glycerol with an unsaturated acid such as (meth)acrylic acid, maleic acid, and fumaric acid; bisacrylamides such as N,N-methylenebisacrylamide; di(meth)acrylic acid esters or tri(meth)acrylic acid esters obtained by reacting a polyepoxide with (meth)acrylic acid; di(meth)acrylic acid carbamyl esters obtained by reacting polyisocyanate such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds such as diglycidyl compounds such as (poly)ethylene glycol diglycidyl ether, (poly)propylene glycol diglycidyl ether, and (poly)glycerol diglycidyl ether and triglycidyl compounds; epihalohydrin compounds such as epichlorohydrin, epibromohydrin, and $\alpha$-methylepichlorohydrin; compounds having two or more reactive functional groups such as isocyanate compounds such as 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal crosslinking agents, a polyglycidyl compound is preferably used, a diglycidyl ether compound is more preferably used, and (poly)ethylene glycol diglycidyl ether, (poly)propylene glycol diglycidyl ether, and (poly)glycerol diglycidyl ether are preferably used. These internal crosslinking agents may be used singly or in combination of two or more.

The amount of the internal crosslinking agent used is preferably 0.000001 to 0.02 mol, more preferably 0.00001 to 0.01 mol, still more preferably 0.00001 to 0.005 mol, and yet still more preferably 0.00005 to 0.002 mol, with respect to 1 mol of the water-soluble ethylenically unsaturated monomer.

[Hydrocarbon Dispersion Medium]

Examples of the hydrocarbon dispersion medium include aliphatic hydrocarbons having six to eight carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are particularly preferably used because they are industrially easily available, have stable quality, and are inexpensive. These hydrocarbon dispersion media may be used singly or in combination of two or more. As an example of a mixture of the hydrocarbon dispersion media, a suitable result can be obtained by using a commercially available product such as Exxsol Heptane (manufactured by Exxon Mobil Corporation: containing 75 to 85 mass% of heptane and an isomer hydrocarbon thereof).

The amount of the hydrocarbon dispersion medium used is preferably 100 to 1,500 parts by mass, and more preferably 200 to 1,400 parts by mass, with respect to 100 parts by mass of the first-stage water-soluble ethylenically unsaturated monomer from the viewpoint of uniformly dispersing the water-soluble ethylenically unsaturated monomer and facilitating the control of the polymerization temperature. As will be described later, the reversed phase suspension polymerization is performed in one stage (single stage) or multiple stages of two or more stages, and the above-described first-stage polymerization means the first-stage polymerization reaction in the single-stage polymerization or the multi-stage polymerization (the same applies hereinafter).

[Dispersion Stabilizer]

(Surfactant)

In the reversed phase suspension polymerization, a dispersion stabilizer can also be used in order to improve the dispersion stability of the water-soluble ethylenically unsaturated monomer in the hydrocarbon dispersion medium. As the dispersion stabilizer, a surfactant can be used.

Examples of the surfactant used include a sucrose fatty acid ester, a polyglycerol fatty acid ester, a sorbitan fatty acid ester, a polyoxyethylene sorbitan fatty acid ester, a polyoxyethylene glycerol fatty acid ester, a sorbitol fatty acid ester, a polyoxyethylene sorbitol fatty acid ester, a polyoxyethylene alkyl ether, a polyoxyethylene alkyl phenyl ether, a polyoxyethylene castor oil, a polyoxyethylene hydrogenated castor oil, an alkylallyl formaldehyde condensed polyoxyethylene ether, a polyoxyethylene polyoxypropylene block copolymer, a polyoxyethylene polyoxypropyl alkyl ether, a polyethylene glycol fatty acid ester, an alkyl glucoside, an N-alkyl gluconamide, a polyoxyethylene fatty acid amide, a polyoxyethylene alkylamine, a phosphoric acid ester of a polyoxyethylene alkyl ether, and a phosphoric acid ester of a polyoxyethylene alkyl allyl ether. Among these surfactants, it is particularly preferable to use a sorbitan fatty acid ester, a polyglycerol fatty acid ester, or a sucrose fatty acid ester from the viewpoint of the dispersion stability of the monomer. These surfactants may be used singly or in combination of two or more.

The amount of the surfactant used is preferably 0.1 to 30 parts by mass, and more preferably 0.3 to 20 parts by mass, with respect to 100 parts by mass of the first-stage water-soluble ethylenically unsaturated monomer.

(Polymeric Dispersant)

As the dispersion stabilizer used in the reversed phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymer, maleic anhydride-modified EPDM (ethylene-propylenediene terpolymer), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, and ethyl hydroxyethyl cellulose. Among these polymeric dispersants, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are particularly preferably used from the viewpoint of the dispersion stability of the monomer. These polymeric dispersants may be used singly or in combination of two or more.

The amount of the polymeric dispersant used is preferably 0.1 to 30 parts by mass, and more preferably 0.3 to 20 parts by mass, with respect to 100 parts by mass of the first-stage water-soluble ethylenically unsaturated monomer.

[Other Components]

In the method for producing water-absorbent resin particles, if desired, other components may be added to an aqueous solution containing the water-soluble ethylenically unsaturated monomer to perform reversed phase suspension polymerization. As other components, various additives such as a thickener and a chain transfer agent can be added.

As an example, reversed phase suspension polymerization can be performed with a thickener added to an aqueous solution containing the water-soluble ethylenically unsaturated monomer. Adding a thickener as described above to adjust the viscosity of the aqueous solution enables the control of the median particle diameter obtained in the reversed phase suspension polymerization.

As the thickener, for example, hydroxyethyl cellulose, hydroxypropyl cellulose, methyl cellulose, carboxymethyl cellulose, polyacrylic acid, a (partially) neutralized polyacrylic acid, polyethylene glycol, polyacrylamide, polyethyleneimine, dextrin, sodium alginate, polyvinyl alcohol, polyvinyl pyrrolidone, or polyethylene oxide or the like can be used. When the stirring rate during polymerization is constant, the primary particles and/or the secondary particles of the resulting particles tend to be larger as the viscosity of the aqueous solution of the water-soluble ethylenically unsaturated monomer is higher.

[Reversed Phase Suspension Polymerization]

In performing the reversed phase suspension polymerization, for example, a monomer aqueous solution containing the water-soluble ethylenically unsaturated monomer is dispersed in a hydrocarbon dispersion medium in the presence of a dispersion stabilizer. At this time, before the polymerization reaction is started, the addition timing of the dispersion stabilizer (surfactant or polymeric dispersant) may be either before or after the addition of the monomer aqueous solution.

Among them, from the viewpoint of easily reducing the amount of the hydrocarbon dispersion medium remaining in the resulting water-absorbent resin particles, it is preferable to perform polymerization after dispersing the surfactant in the hydrocarbon dispersion medium in which the polymeric dispersant has been dispersed and in which the monomer aqueous solution has then been dispersed.

Such reversed phase suspension polymerization can be performed in one stage or multiple stages of two or more stages. From the viewpoint of enhancing productivity, it is preferable to employ two or three stages.

When the reversed phase suspension polymerization is performed in two or more stages, the first-stage reversed phase suspension polymerization is performed, then the water-soluble ethylenically unsaturated monomer is added to and mixed with the reaction mixture obtained in the first-stage polymerization reaction, and the second-stage or subsequent reversed phase suspension polymerization may be performed in the same manner as the first-stage polymerization. In the reversed phase suspension polymerization in each of the second and subsequent stages, in addition to the water-soluble ethylenically unsaturated monomer, a radical polymerization initiator is preferably added within the range of the molar ratio of each component to the water-soluble ethylenically unsaturated monomer described above based on the amount of the water-soluble ethylenically unsaturated monomer added in the reversed phase suspension polymerization in each of the second and subsequent stages to perform reversed phase suspension polymerization. In the second and subsequent polymerization, an internal crosslinking agent may be added to the water-soluble ethylenically unsaturated monomer as necessary.

The reaction temperature of the polymerization reaction is preferably 20 to 110°C, and more preferably 40 to 90°C, from the viewpoint of enhancing the economic efficiency by rapidly progressing the polymerization and shortening the polymerization time and easily removing the heat of polymerization to smoothly perform the reaction.

<Surface Crosslinking Step>

Next, the water-absorbent resin particles of the present invention are obtained by adding a surface crosslinking agent to a hydrous gel having an internally crosslinked structure obtained by polymerizing a water-soluble ethylenically unsaturated monomer to crosslink the hydrous gel (surface crosslinking reaction). This surface crosslinking reaction is preferably performed in the presence of a surface crosslinking agent after polymerization of the water-soluble ethylenically unsaturated monomer. As described above, by subjecting the hydrous gel having an internally crosslinked structure to a surface crosslinking reaction after polymerization, it is possible to obtain water-absorbent resin particles in which the crosslinking density in the vicinity of the surface of the water-absorbent resin particles is increased and various performances such as water absorption capacity under a load are improved.

Examples of the surface crosslinking agent include compounds having two or more reactive functional groups. Examples include polyols such as ethylene glycol, propylene glycol, 1,4-butanediol, diethylene glycol, triethylene glycol, trimethylolpropane, glycerol, polyoxyethylene glycol, polyoxypropylene glycol, and polyglycerol; polyglycidyl compounds such as (poly)ethylene glycol diglycidyl ether, (poly)glycerol diglycidyl ether, (poly)glycerol triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylenebisoxazoline; carbonate compounds such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one (for example, alkylene carbonate); and hydroxyalkylamide compounds such as bis[N,N-di($\beta$-hydroxyethyl)]adipamide. Among these surface crosslinking agents, polyglycidyl compounds such as (poly)ethylene glycol diglycidyl ether, (poly)glycerol diglycidyl ether, (poly)glycerol triglycidyl ether, trimethylolpropane triglycidyl ether, (poly)propylene glycol polyglycidyl ether, and (poly)glycerol polyglycidyl ether; and carbonate compounds (for example, alkylene carbonate) such as ethylene carbonate, propylene carbonate, 4,5-dimethyl-1,3-dioxolane-2-one, 4,4-dimethyl-1,3-dioxolane-2-one, 4-ethyl-1,3-dioxolane-2-one, 4-hydroxymethyl-1,3-dioxolane-2-one, 1,3-dioxane-2-one, 4-methyl-1,3-dioxane-2-one, 4,6-dimethyl-1,3-dioxane-2-one, and 1,3-dioxopane-2-one are preferable. These surface crosslinking agents may be used singly or in combination of two or more.

The amount of the surface crosslinking agent used is preferably 0.00001 to 0.01 mol, more preferably 0.00005 to 0.005 mol, and still more preferably 0.0001 to 0.002 mol, with respect to a total of 1 mol of the water-soluble ethylenic unsaturated monomer used for the polymerization.

As a method for adding the surface crosslinking agent, the surface crosslinking agent may be added as it is or as an aqueous solution, or may be added as a solution containing a hydrophilic organic solvent as a solvent as necessary. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used singly or in combination of two or more or may be used as a mixed solvent with water.

The addition timing of the surface crosslinking agent may be after almost all the polymerization reaction of the water-soluble ethylenically unsaturated monomer is completed, and the surface crosslinking agent is preferably added in the presence of water in the range of 1 to 400 parts by mass, more preferably in the presence of water in the range of 5 to 200 parts by mass, still more preferably in the presence of water in the range of 10 to 100 parts by mass, and yet still more preferably in the presence of water in the range of 20 to 60 parts by mass, with respect to 100 parts by mass of the water-soluble ethylenically unsaturated monomer. The amount of water means the total amount of water contained in the reaction system and water used as necessary when the surface crosslinking agent is added.

The reaction temperature in the surface crosslinking reaction is preferably 50 to 250°C, more preferably 60 to 180°C, still more preferably 60 to 140°C, and yet still more preferably 70 to 120°C. The reaction time of the surface crosslinking reaction is preferably 1 to 300 minutes, and more preferably 5 to 200 minutes.

<Drying Step>

The method may include a drying step of removing water, a hydrocarbon dispersion medium, and the like by distillation by externally applying energy such as heat after performing the reversed phase suspension polymerization described above. When dehydration is performed from the hydrous gel after the reversed phase suspension polymerization, the system in which the hydrous gel is dispersed in the hydrocarbon dispersion medium is heated, so that water and the hydrocarbon dispersion medium are temporarily distilled off to the outside of the system by azeotropic distillation. At this time, when only the distilled hydrocarbon dispersion medium is returned into the system, continuous azeotropic distillation becomes possible. In that case, since the temperature in the system during drying is maintained at an azeotropic temperature with the hydrocarbon dispersion medium or lower, that case is preferable from the viewpoint that the resin is hardly deteriorated. Subsequently, water and the hydrocarbon dispersion medium are distilled off to obtain the water-absorbent resin particles. Various performances of the water-absorbent resin particles to be obtained can be controlled by controlling the treatment conditions in the drying step after the polymerization to adjust the amount of water to be removed.

In the drying step, the drying treatment by distillation may be performed under normal pressure or under reduced pressure. From the viewpoint of enhancing the drying efficiency, the drying may be performed under a flow of nitrogen or the like. The drying temperature when the drying treatment is performed under normal pressure is preferably 70 to 250°C, more preferably 80 to 180°C, still more preferably 80 to 140°C, and yet still more preferably 90 to 130°C. When the drying treatment is performed under reduced pressure, the drying temperature is preferably 40 to 160°C, and more preferably 50 to 110°C.

The water content of the water-absorbent resin particles obtained by the production method of the present invention is preferably 20 mass% or less, more preferably 15 mass% or less, and still more preferably 10 mass% or less. The range of the water content is preferably 1 to 10 mass% or the like.

When the surface crosslinking step with the surface crosslinking agent is performed after the polymerization of the monomer is performed by reversed phase suspension polymerization, the drying step by distillation described above is performed after the completion of the surface crosslinking step. Alternatively, the surface crosslinking step and the drying step may be performed simultaneously.

The water-absorbing resin composition of the present invention may contain an additive according to the purpose. Examples of such additives include an inorganic powder, a surfactant, an oxidant, a reducing agent, a radical chain inhibitor, an antioxidant, and an antibacterial agent. For example, the flowability of the water-absorbing resin composition can be further improved by adding 0.05 to 5 parts by mass of amorphous silica as an inorganic powder to 100 parts by mass of the water-absorbent resin particles. The additive is preferably hydrophilic or water-soluble.

In the water-absorbing resin composition of the present invention, the content of the water-absorbent resin particles (excluding the additives) is preferably 70 mass% or more, more preferably 80 mass% or more, and still more preferably 90 mass% or more.

From the viewpoint of more suitably exhibiting the effect of the present invention, the physiological saline retention amount of the water-absorbing resin composition is preferably 25 g/g or more, and more preferably 30 g/g or more, and is preferably 60 g/g or less, more preferably 55 *g/g* or less, and still more preferably 50 *g/g* or less. The range of the physiological saline retention amount is preferably 25 to 60 g/g or 30 to 50 *g/g* or the like.

From the viewpoint of more suitably exhibiting the effect of the present invention, the physiological saline absorption amount under a load of 4.14 kPa of the water-absorbing resin composition is preferably 5 ml/g or more, more preferably 10 ml/g or more, and still more preferably 15 ml/g or more, and is preferably 40 ml/g or less, more preferably 35 ml/g or less, and still more preferably 30 ml/g or less. The range of the physiological saline absorption amount is preferably 5 to 40 ml/g or 10 to 30 ml/g or the like.

Each of the physiological saline retention amount and the physiological saline absorption amount under a load of 4.14 kPa of the water-absorbing resin composition is a value measured by the method described in Examples.

From the viewpoint of more suitably exerting the effect of the present invention, the water absorption rate by the Vortex method of the water-absorbing resin composition is preferably 60 seconds or less, more preferably 50 seconds or less, and still more preferably 40 seconds or less, and is preferably 1 second or more, more preferably 3 seconds or more, and still more preferably 10 seconds or more. The range of the water absorption rate is preferably 1 to 60 seconds or 3 to 40 seconds or the like.

The water absorption rate of the water-absorbing resin composition by the Vortex method is a value measured by the method described in Examples.

In the water-absorbing resin composition of the present invention, the "soluble component eluted for 1 hour after the deterioration test" evaluated by the method described in Examples is preferably 20 mass% or less, and more preferably 14 mass% or less, and is preferably 0 mass% or more, and more preferably 1 mass% or more. The range of the soluble component is preferably 0 to 20 mass% or 1 to 14 mass% or the like.

In the water-absorbing resin composition of the present invention, the "initial value of gel strength" evaluated by the method described in Examples is preferably 5,000 N/m$^2$ or more, more preferably 6,000 N/m$^2$ or more, and still more preferably 6,500 N/m$^2$ or more, from the viewpoint of gel stability when the hygienic material is usually worn. The upper limit of the initial value of the gel strength is, for example, 20,000 N/m$^2$.

In the water-absorbing resin composition of the present invention, the "gel strength after being allowed to stand at 37°C for 14 hours" evaluated by the method described in Examples is preferably 4,000 N/m$^2$ or more, more preferably 4,500 N/m$^2$ or more, and still more preferably 5,000 N/m$^2$ or more, from the viewpoint of gel stability when the hygienic material is usually worn. The upper limit of the gel strength is, for example, 20,000 N/m$^2$.

In the water-absorbing resin composition of the present invention, the "gel strength change degree" evaluated by the method described in Examples is preferably 40% or less, more preferably 35% or less, and still more preferably 30% or less, from the viewpoint of gel stability when the hygienic material is usually worn. The lower limit of the gel strength change degree is, for example, 0%.

In the water-absorbing resin composition of the present invention, the "gel strength after being allowed to stand at 37°C for 14 hours and further irradiated with ultraviolet ray", evaluated by the method described in the Examples is preferably 7,000 N/m$^2$ or less, more preferably 6,000 N/m$^2$ or less, and still more preferably 4,000 N/m$^2$ or less, and is preferably 0 N/m$^2$ or more, more preferably 500 N/m$^2$ or more, and more preferably 1,000 N/m$^2$ or more, from the viewpoint of handleability when the water-absorbing resin composition is separated from the hygienic material after being irradiated with ultraviolet rays as a waste treatment means.

In the water-absorbing resin composition of the present invention, the "gel strength change degree after UV irradiation" evaluated by the method described in Examples is preferably 40% or more, more preferably 45% or more, and still more preferably 50% or more, and is preferably 100% or less, more preferably 95% or less, and still more preferably 90% or less, from the viewpoint of handleability when the water-absorbing resin composition is separated from the hygienic material after being irradiated with ultraviolet rays as a waste treatment means. The range of the gel strength change degree is preferably 40 to 100 mass% or 40 to 90 mass% or the like.

The method for producing the water-absorbing resin composition of the present invention is not particularly limited, but the water-absorbing resin composition can be suitably produced by a method for producing a water-absorbing resin composition including a step of mixing the water-absorbent resin particles before surface-crosslinking with the chelating agent and/or a step of mixing the water-absorbent resin particles after surface-crosslinking with the chelating agent. That is, the step of mixing the water-absorbent resin particles and the chelating agent may be a step of mixing the water-absorbent resin particles before surface-crosslinking with the chelating agent and then surface-crosslinking the water-absorbent resin particles, a step of mixing the water-absorbent resin particles with the chelating agent after surface-crosslinking the water-absorbent resin particles, or a step of mixing the water-absorbent resin particles before surface-crosslinking with the chelating agent, then surface-crosslinking the water-absorbent resin particles, and further mixing the water-absorbent resin particles after surface-crosslinking with the chelating agent. The water-absorbent resin particles and the chelating agent are as described above.

As described above, the water-absorbent resin particles and the chelating agent can be suitably mixed by, for example, mixing them in a solid phase state.

2. Absorber and Absorbent Article

The water-absorbing resin composition of the present invention constitutes an absorber used for hygienic materials such as sanitary products and disposable diapers and is suitably used for an absorbent article including the absorber.

Here, the absorber using the water-absorbing resin composition of the present invention includes the water-absorbing resin composition of the present invention. The absorber may further contain hydrophilic fibers. Examples of the configuration of the absorber include a sheet-like structure in which the water-absorbing resin composition is fixed on a nonwoven fabric or between a plurality of nonwoven fabrics, a mixed dispersion obtained by mixing the water-absorbing resin composition and hydrophilic fibers so as to have a uniform composition, a sandwich structure in which the water-absorbing resin composition is sandwiched between layered hydrophilic fibers, and a structure in which the water-absorbing resin composition and hydrophilic fibers are wrapped with tissue. The absorber may contain other components, for example, an adhesive binder such as a heat-sealable synthetic fiber, a hot melt adhesive, or an adhesive emulsion for enhancing the shape retention of the absorber.

The content of the water-absorbing resin composition in the absorber is preferably 5 to 100 mass%, more preferably 10 to 95 mass%, still more preferably 20 to 90 mass%, and yet still more preferably 30 to 80 mass%.

Examples of the hydrophilic fibers include cellulose fibers such as cotton-like pulp, mechanical pulp, chemical pulp, and semi-chemical pulp obtained from wood, artificial cellulose fibers such as rayon and acetate, and fibers made of synthetic resins such as hydrophilized polyamide, polyester, and polyolefin. The average fiber length of the hydrophilic fibers is usually 0.1 to 10 mm or may be 0.5 to 5 mm.

The absorber using the water-absorbing resin composition of the present invention can be held between a liquid-permeable sheet (top sheet) through which a liquid can pass and a liquid-impermeable sheet (back sheet) through which a liquid cannot pass to form the absorbent article of the present invention. The liquid-permeable sheet is disposed on the side in contact with the body, and the liquid-impermeable sheet is disposed on the side opposite to the side in contact with the body.

Examples of the liquid-permeable sheet include nonwoven fabrics of an air-through type, a spunbond type, a chemical bond type, and a needle punch type and the like made of fibers such as polyethylene, polypropylene, and polyester, and porous synthetic resin sheets. Examples of the liquid-impermeable sheet include synthetic resin films made of resins such as polyethylene, polypropylene, and polyvinyl chloride. Examples

The present invention will be hereinafter described in detail with reference to Examples and Comparative Examples. However, the present invention is not limited to Examples.

Water-absorbent resin particles obtained in the following Production Examples and water-absorbing resin compositions obtained in Examples and Comparative Examples were evaluated in the following various tests. Unless otherwise specified, the measurement was performed under an environment of a temperature of 25±2°C and a humidity of 50±10%.

[Production of Water-Absorbent Resin Particles]

<Production Example 1>

Prepared was a 2 L round-bottomed cylindrical separable flask having an inner diameter of 11 cm and equipped with a reflux condenser, a dropping funnel, a nitrogen gas introduction tube, and a stirring blade having two stages of 4-inclined paddle blades having a blade diameter of 5 cm as a stirrer. To this flask, 293 g of n-heptane as a hydrocarbon dispersion medium and 0.736 g of a maleic anhydride-modified ethylene-propylene copolymer (Mitsui Chemicals, Inc., Hi-wax 1105A) as a polymeric dispersant were added, and the temperature was raised to 80°C with stirring to dissolve the dispersant, followed by cooling to 50°C. Meanwhile, 92.0 g (1.03 mol) of an 80.5 mass% aqueous solution of acrylic acid as a water-soluble ethylenically unsaturated monomer was placed in a beaker having an internal volume of 300 mL, 147.7 g of a 20.9 mass% aqueous solution of sodium hydroxide was added dropwise with cooling with ice water to perform neutralization of 75 mol%, and then 0.092 g of hydroxyl ethyl cellulose (Sumitomo Seika Chemicals Company, Limited, HEC AW-15F) as a thickener, 0.0736 g (0.272 mmol) of potassium persulfate as a water-soluble radical polymerization agent, and 0.010 g (0.057 mmol) of ethylene glycol diglycidyl ether as an internal crosslinking agent were added thereto and dissolved, thereby preparing a first-stage aqueous solution. The aqueous liquid prepared above was added to a separable flask and stirred for 10 minutes, and then a surfactant solution obtained by heating and dissolving 0.736 g of a sucrose stearate of HLB3 (Ryoto sugar ester S-370 manufactured by Mitsubishi Chemical Foods Corporation) as a surfactant in 6.62 g of n-heptane was further added in a 20 mL-vial. While the rotation speed of the stirrer was set to 550 rpm and stirring, the inside of the system was sufficiently replaced with nitrogen. Thereafter, the flask was immersed in a water bath at 70°C and heated, and polymerization was performed for 60 minutes to obtain a first-stage polymerization slurry liquid.

Meanwhile, 128.8 g (1.43 mol) of an 80.5 mass% aqueous solution of acrylic acid as a water-soluble ethylenically unsaturated monomer was placed in another beaker having an internal volume of 500 mL, and 159.0 g of a 27 mass% aqueous solution of sodium hydroxide was added dropwise while cooling with ice water to perform neutralization of 75 mol%. Then, 0.103 g (0.381 mmol) of potassium persulfate as a water-soluble radical polymerization initiator and 0.0116 g (0.067 mmol) of ethylene glycol diglycidyl ether as an internal crosslinking agent were added thereto and dissolved, thereby preparing a second-stage aqueous liquid.

The inside of the separable flask system was cooled to 27°C while the rotation speed of the stirrer was set to 1000 rpm, and then the whole amount of the second-stage aqueous liquid was added to the first-stage polymerization slurry liquid, and the inside of the system was replaced with nitrogen for 30 minutes. Then, the flask was immersed in a water bath at 70°C again, the temperature was raised, and the polymerization reaction was performed for 60 minutes to obtain a hydrous gel polymer.

Thereafter, the flask was immersed in an oil bath set at 125°C, and 251.7 g of water was removed to the outside of the system while refluxing n-heptane by azeotropic distillation of n-heptane and water. Thereafter, 4.42 g (0.507 mmol) of a 2 mass% aqueous solution of ethylene glycol diglycidyl ether as a surface crosslinking agent was added to the flask, and the mixture was held at 83°C for 2 hours.

Thereafter, n-heptane was evaporated at 125°C for drying, and further passed through a sieve with a mesh size of 850 $\mu$m to obtain 220.2 g of water-absorbent resin particles (1). The water-absorbent resin particles (1) had a physiological saline retention amount of 40 g/g, a physiological saline absorption amount under a load of 4.14 kPa of 15 mL/g, a water content of 8 mass%, and a median particle diameter of 370 $\mu$m.

[Production of Water-Absorbing Resin Composition]

<Example 1>

To 100 parts by mass of the water-absorbent resin particles (1) obtained in Production Example 1, 0.1 parts by mass of ethylenediamine-N, N'-disuccinic acid (EDDS·4H, iron (III) chelate stability constant: 20.1 [logKML], the number of carboxyl groups in the molecule: 4) as a chelating agent was added in the form of a powder, followed by mixing for 30 minutes (condition: revolution speed 50 rpm, rotation speed 50 rpm) using a cross rotary mixer manufactured by Meiwa Kogyo Co., Ltd., and 0.5 parts by mass of amorphous silica (Oriental Silicas Corporation, TOKUSIL NP-S) was further mixed with respect to 100 parts by mass of the water-absorbent resin particles (1) to obtain a water-absorbing resin composition (1).

<Example 2>

20 g of the water-absorbent resin particles (1) obtained in Production Example 1 were weighed into a round-bottom cylindrical separable flask having an inner diameter of 11 cm and equipped with an anchor type stirring blade made of a fluororesin. Next, while stirring at 300 rpm, a chelating agent aqueous solution prepared by adding 0.143 g of ion-exchanged water to 0.057 g of a 35 mass% aqueous solution of ethylenediamine-N,N'-disuccinic acid trisodium (EDDS·3Na, iron (III) chelate stability constant: 20.1 [logKML], the number of carboxyl groups in the molecule: 4) was added dropwise to a separable flask with a Pasteur pipette, followed by stirring for 10 minutes to obtain a mixture. This mixture was heated at 100°C for 30 minutes, and then 0.5 parts by mass of amorphous silica (Oriental Silicas Corporation, TOKUSIL NP-S) was further mixed with respect to 100 parts by mass of the water-absorbent resin particles (1) to obtain a water-absorbing resin composition (2).

<Example 3>

A water-absorbing resin composition (3) was obtained by performing the same operations as those of Example 1 except using 0.1 parts by mass of glycol ether diamine tetraacetic acid (GEDTA, iron (III) chelate stability constant: 20.5 [logKML], the number of carboxyl groups in the molecule: 4) instead of using 0.1 parts by mass of ethylenediamine-N,N'-disuccinic acid (EDDS·4H) in Example 1.

<Comparative Example 1>

A water-absorbing resin composition (4) was obtained by performing the same operations as those of Example 2 except using a chelating agent aqueous solution prepared by using 0.044 g of a 45 mass% aqueous solution of diethylenetriamine pentaacetic acid·pentasodium (DTPA·5Na, iron (III) chelate stability constant: 28.6 [logKML], the number of carboxyl groups in the molecule: 5) and changing the amount of ion-exchanged water from 0.143 g to 0.156 g instead of using 0.057 g of the 35 mass% aqueous solution of ethylenediamine-N,N'-disuccinic acidtrisodium (EDDS·3Na) in Example 2.

<Comparative Example 2>

A water-absorbing resin composition (5) was obtained by performing the same operations as those of Example 1 except using 0.1 parts by mass of ethylenediaminetetramethylenephosphonic acid dihydrate (EDTMP·2H$_2$O, iron (III) chelate stability constant: 19.6 [logKML], the number of carboxyl groups in the molecule: 0) instead of using 0.1 parts by mass of ethylenediamine-N,N'-disuccinic acid (EDDS·4H) in Example 1.

<Comparative Example 3>

A water-absorbing resin composition (6) was obtained by performing the same operations as those of Example 1 except using 0.1 parts by mass of hydroxyethylenediaminetriacetic acid (EDTA-OH, iron (III) chelate stability constant: 19.8 [logKML], the number of carboxyl groups in the molecule: 3) instead of using 0.1 parts by mass of ethylenediamine-N,N'-disuccinic acid (EDDS·4H) in Example 1.

<Comparative Example 4>

A water-absorbing resin composition (7) was obtained by performing the same operations as those of Example 2 except using a chelating agent aqueous solution prepared by using 0.050 g of 40 mass% aqueous solution of N-methylglycine diacetic acid trisodium (MGDA·3Na, iron (III) chelate stability constant: 16.5 [logKML], the number of carboxyl groups in the molecule: 3) and changing the amount of ion-exchanged water from 0.143 g to 0.150 g instead of using 0.057 g of the 35 mass% aqueous solution of ethylenediamine-N,N'-disuccinic acid trisodium (EDDS·3Na) in Example 2.

<Comparative Example 5>

A water-absorbing resin composition (8) was obtained by performing the same operations as those of Example 2 except using a chelating agent aqueous solution prepared by using 0.043 g of a 47 mass% aqueous solution of L-glutamic acid-diacetic acid tetrasodium (GLDA·4Na, iron (III) chelate stability constant: 15.2 [logKML], the number of carboxyl groups in the molecule: 4) and changing the amount of ion-exchanged water from 0.143 g to 0.157 g instead of using 0.057 g of the 35 mass% aqueous solution of ethylenediamine-N,N'-disuccinic acid trisodium (EDDS·3Na) in Example 2.

<Comparative Example 6>

A water-absorbing resin composition (9) was obtained by performing the same operations as those of Example 1 except using 0.1 parts by mass of iminodisuccinic acid (IDS, iron (III) chelate stability constant: 13.9 [logKML], the number of carboxyl groups in the molecule: 4) instead of using 0.1 parts by mass of ethylenediamine-N,N'-disuccinic acid (EDDS·4H) in Example 1.

<Comparative Example 7>

A water-absorbing resin composition (10) was obtained by performing the same operations as those of Example 2 except using a chelating agent aqueous solution prepared by using 0.050 g of a 40 mass% polyaspartic acid sodium (molecular weight: 2,000 to 3,000, iron (III) chelate stability constant: 18.5 [logKML], the number of carboxyl groups in the molecule: 14 or more) aqueous solution and changing the amount of ion-exchanged water from 0.143 g to 0.150 g instead of using 0.057 g of the 35 mass% ethylenediamine-N,N'-disuccinic acid trisodium (EDDS·3Na) aqueous solution in Example 2.

<Comparative Example 8>

A water-absorbing resin composition (11) was obtained by performing the same operations as those of Example 2 except using a chelating agent aqueous solution prepared by using 0.040 g of a 50 mass% aqueous solution of 3-hydroxy-2,2'-iminodisuccinic acid tetrasodium (HIDS, iron (III) chelate stability constant: 15.0 [logKML], the number of carboxyl groups in the molecule: 4) and changing the amount of ion-exchanged water from 0.143 g to 0.160 g instead of using 0.057 g of the 35 mass% aqueous solution of ethylenediamine-N,N'-disuccinic acidtrisodium (EDDS·3Na) in Example 2.

<Comparative Example 9>

A water-absorbing resin composition (12) was obtained by performing the same operations as those of Example 1 except using 0.1 parts by mass of L(+)-tartaric acid disodium dihydrate (iron (III) chelate stability constant: 18.1 [logKML], the number of carboxyl groups in the molecule: 2) instead of using 0.1 parts by mass of ethylenediamine-N,N'-disuccinic acid (EDDS·4H) in Example 1.

<Comparative Example 10>

A water-absorbing resin composition (13) was obtained by performing the same operations as those of Example 1 except using 0.1 parts by mass of citric acid trisodium dihydrate (iron (III) chelate stability constant: 11.9 [logKML], the number of carboxyl groups in the molecule: 3) instead of using 0.1 parts by mass of ethylenediamine-N,N'-disuccinic acid (EDDS·4H) in Example 1.

<Comparative Example 11>

A water-absorbing resin composition (14) was obtained by performing the same operations as those of Example 1 except using 0.1 parts by mass of pyridine-2,6-dicarboxylic acid (iron (III) chelate stability constant: 10.9 [logKML], the number of carboxyl groups in the molecule: 2) instead of using 0.1 parts by mass of ethylenediamine-N,N'-disuccinic acid (EDDS·4H) in Example 1.

<Comparative Example 12>

A water-absorbing resin composition (15) was obtained by performing the same operations as those of Example 1 except using 0.1 parts by mass of ethylenediaminetetraacetic acid (EDTA, iron (III) chelate stability constant: 25.1 [logKML], the number of carboxyl groups in the molecule: 4) instead of 0.1 parts by mass of ethylenediamine-N,N'-disuccinic acid (EDDS·4H) in Example 1.

<Comparative Example 13>

A water-absorbing resin composition (16) was obtained by performing the same operations as those of Example 1 except using 0.1 parts by mass of triethylenetetraminehexaacetic acid (TTHA, iron (III) chelate stability constant: 26.8 [logKML], the number of carboxyl groups in the molecule: 6) instead of using 0.1 parts by mass of ethylenediamine-N,N'-disuccinic acid (EDDS·4H) in Example 1.

<Comparative Example 14>

A water-absorbing resin composition (17) was obtained by performing the same operations as those of Example 1 except using 0.1 parts by mass of gluconic acid sodium (iron (III) chelate stability constant: 37.2 [logKML], the number of carboxyl groups in the molecule: 1) instead of using 0.1 parts by mass of ethylenediamine-N,N'-disuccinic acid (EDDS·4H) in Example 1.

<Comparative Example 15>

A water-absorbing resin composition (18) was obtained by performing the same operations as those of Example 1 except using 0.1 parts by mass of glucoheptonic acid sodium dihydrate (iron (III) chelate stability constant: 38.3 [logKML], the number of carboxyl groups in the molecule: 1) instead of using 0.1 parts by mass of ethylenediamine-N,N'-disuccinic acid (EDDS·4H) in Example 1.

<Example 4>

A water-absorbing resin composition (19) was obtained by performing the same operations as those of Example 2 except changing the amount of a 35 mass% aqueous solution of ethylenediamine-N,N'-disuccinic acid trisodium (EDDS·3Na) to 0.2 g as a chelating agent aqueous solution without using ion-exchanged water in Example 2.

<Example 5>

Prepared was a 2 L round-bottomed cylindrical separable flask having an inner diameter of 11 cm and equipped with a reflux condenser, a dropping funnel, a nitrogen gas introduction tube, and a stirring blade having two stages of 4-inclined paddle blades having a blade diameter of 5 cm as a stirrer. To this flask, 293 g of n-heptane as a hydrocarbon dispersion medium and 0.736 g of a maleic anhydride-modified ethylene-propylene copolymer (Mitsui Chemicals, Inc., Hi-wax 1105A) as a polymeric dispersant were added, and the temperature was raised to 80°C with stirring to dissolve the dispersant, followed by cooling to 50°C. Meanwhile, 92.0 g (1.03 mol) of an 80.5 mass% aqueous solution of acrylic acid as a water-soluble ethylenically unsaturated monomer was placed in a beaker having an internal volume of 300 mL, 147.7 g of a 20.9 mass% aqueous solution of sodium hydroxide was added dropwise with cooling with ice water to perform neutralization of 75 mol%, and then 0.092 g of hydroxyl ethyl cellulose (Sumitomo Seika Chemicals Company, Limited, HEC AW-15F) as a thickener, 0.0736 g (0.272 mmol) of potassium persulfate as a water-soluble radical polymerization agent, and 0.010 g (0.057 mmol) of ethylene glycol diglycidyl ether as an internal crosslinking agent were added thereto and dissolved, thereby preparing a first-stage aqueous solution. The aqueous liquid prepared above was added to a separable flask and stirred for 10 minutes, and then a surfactant solution obtained by heating and dissolving 0.736 g of a sucrose stearate of HLB3 (Ryoto sugar ester S-370 manufactured by Mitsubishi Chemical Foods Corporation) as a surfactant in 6.62 g of n-heptane was further added in a 20 mL-vial. While the rotation speed of the stirrer was set to 550 rpm and stirring, the inside of the system was sufficiently replaced with nitrogen. Thereafter, the flask was immersed in a water bath at 70°C and heated, and polymerization was performed for 60 minutes to obtain a first-stage polymerization slurry liquid.

Meanwhile, 128.8 g (1.43 mol) of an 80.5 mass% aqueous solution of acrylic acid as a water-soluble ethylenically unsaturated monomer was placed in another beaker having an internal volume of 500 mL, and 159.0 g of a 27 mass% aqueous solution of sodium hydroxide was added dropwise while cooling with ice water to perform neutralization of 75 mol%. Then, 0.103 g (0.381 mmol) of potassium persulfate as a water-soluble radical polymerization initiator and 0.0116 g (0.067 mmol) of ethylene glycol diglycidyl ether as an internal crosslinking agent were added thereto and dissolved, thereby preparing a second-stage aqueous liquid.

The inside of the separable flask system was cooled to 27°C while the rotation speed of the stirrer was set to 1000 rpm, and then the whole amount of the second-stage aqueous liquid was added to the first-stage polymerization slurry liquid, and the inside of the system was replaced with nitrogen for 30 minutes. Then, the flask was immersed in a water bath at 70°C again, the temperature was raised, and the polymerization reaction was performed for 60 minutes to obtain a hydrous gel polymer.

Thereafter, the flask was immersed in an oil bath set at 125°C, and 176.6 g of water was removed to the outside of the system while refluxing n-heptane by azeotropic distillation of n-heptane and water. Thereafter, an aqueous chelating agent solution prepared by adding 1.281 g of ion-exchanged water to 0.189 g of a 35 mass% aqueous solution of ethylenediamine-N,N'-disuccinic acid trisodium was added under stirring. Thereafter, 76.4 g of water was removed to the outside of the system while refluxing n-heptane by azeotropic distillation of n-heptane and water again. Thereafter, 4.42 g (0.507 mmol) of a 2 mass% aqueous solution of ethylene glycol diglycidyl ether as a surface crosslinking agent was added to the flask, and the mixture was held at 83°C for 2 hours.

Thereafter, n-heptane was evaporated at 125°C, dried, and further passed through a sieve with a mesh size of 850 $\mu$m to obtain 225.7 g of water-absorbent resin particles. 0.5 parts by mass of amorphous silica (Oriental Silicas Corporation, TOKUSIL NP-S) was mixed with 100 parts by mass of the obtained water-absorbent resin particles to obtain 226.8 g of a water-absorbing resin composition (20). The water-absorbing resin composition (20) had a physiological saline retention amount of 41 g/g, a physiological saline absorption amount under a load of 4.14 kPa of 11 mL/g, a water content of 8 mass%, and a median particle diameter of 361 $\mu$m.

<Example 6>

231.1 g of a water-absorbing resin composition (21) was obtained by performing the same operations as those of Example 5 except changing the amount of 0.189 g of the 35 mass% ethylenediamine-N,N'-disuccinic acid trisodium (EDDS·3Na) aqueous solution added to the hydrous gel polymer of the second stage in Example 5 to 0.063 g, using a chelating agent aqueous solution prepared by changing the amount of ion-exchanged water from 1.281 g to 0.427 g, and changing the amount of water to be removed to the outside of the system by azeotropic distillation of n-heptane and water from 76.4 g to 75.5 g. The water-absorbing resin composition (21) had a physiological saline retention amount of 39 g/g, a physiological saline absorption amount under a load of 4.14 kPa of 19 mL/g, a water content of 9 mass%, and a median particle diameter of 372 $\mu$m.

<Comparative Example 16>

A water-absorbing resin composition (22) was obtained by performing the same operations as those of Example 2 except using a chelating agent aqueous solution prepared by using 0.175 g of 40 mass% N-methylglycine diacetic acid trisodium (MGDA·3Na, iron (III) chelate stability constant: 16.5 [logKML], the number of carboxyl groups in the molecule: 3) and changing the amount of ion-exchanged water from 0.143 g to 0.025 g instead of using 0.057 g of the 35 mass% aqueous solution of ethylenediamine-N,N'-disuccinic acid trisodium (EDDS·3Na) in Example 2.

<Comparative Example 17>

A water-absorbing resin composition (23) was obtained by performing the same operations as those of Comparative Example 16 except changing the amount of a 40 mass% aqueous solution of N-methylglycine diacetic acidtrisodium (MGDA·3Na) to 0.425 g as a chelating agent aqueous solution without using ion-exchanged water in Comparative Example 16.

<Example 7>

227.9 g of a water-absorbing resin composition (24) was obtained by performing the same operations as those of Example 5 except changing the amount of water to be removed to the outside of the system by azeotropic distillation of n-heptane and water from 76.4 g to 65.5 g in Example 5. The water-absorbing resin composition (24) had a physiological saline retention amount of 35 g/g, a physiological saline absorption amount under a load of 4.14 kPa of 28 mL/g, a water content of 7 mass%, and a median particle diameter of 353 $\mu$m.

<Example 8>

229.2 g of a water-absorbing resin composition (25) was obtained by performing the same operations as those of Example 5 except changing the amount of water to be removed to the outside of the system by azeotropic distillation of n-heptane and water from 76.4 g to 78.6 g in Example 5. The water-absorbing resin composition (25) had a physiological saline retention amount of 46 g/g, a physiological saline absorption amount under a load of 4.14 kPa of 10 mL/g, a water content of 8 mass%, and a median particle diameter of 351 $\mu$m.

[Evaluation of Water-Absorbent Resin Particles]

<Physiological Saline Retention Amount>

A cotton bag (Cottonbroad No. 60, 100 mm in width, 200 mm in length) obtained by weighing 2.0 g of the water-absorbent resin particles was placed in a 500-mL beaker. 500 g of a 0.9 mass% aqueous sodium chloride solution (physiological saline) was poured into the cotton bag containing the water-absorbent resin particles at a time so as not to form lumps. The upper portion of the cotton bag was tied with a rubber band, and the cotton bag was allowed to stand for 30 minutes to swell the water-absorbent resin particles. The cotton bag after 30 minutes was dehydrated for 1 minute using a dehydrator (product number: H-122, manufactured by KOKUSAN Co., Ltd.) set so as to have a centrifugal force of 167 G, and the mass Wd (g) of the dehydrated cotton bag containing the swollen gel was measured. The same operation was performed without adding the water-absorbent resin particles, the empty mass We (g) of the cotton bag in a wet state was measured, and the physiological saline retention amount was calculated from the following formula.

$$\text{Physiological saline retention amount (g/g)} = [\text{Wd-We}]/2.0$$

<Physiological Saline Absorption Amount Under Load of 4.14 kPa>

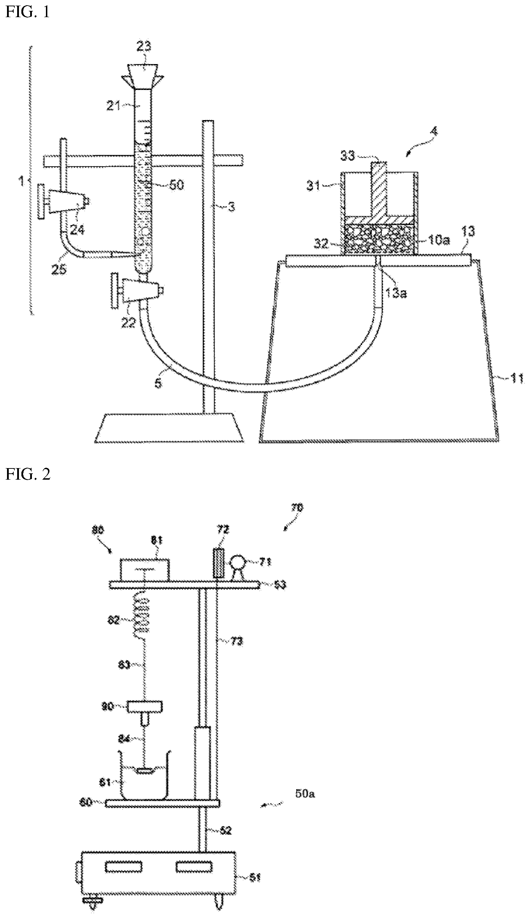

The physiological saline absorption amount (water absorption amount under a load) under a load of 4.14 kPa was measured using a measuring device schematically shown in Fig. 1. The measurement was performed twice for one kind of water-absorbent resin particles, and the average value was obtained. The measuring device includes a burette part 1, a clamp 3, a conduit 5, a frame 11, a measuring table 13, and a measurement part 4 placed on the measuring table 13. The burette part 1 includes a burette tube 21 on which a scale is engraved, a rubber stopper 23 for hermetically sealing the opening of the upper portion of the burette tube 21, a cock 22 connected to the distal end of the lower part of the burette tube 21, and an air introduction pipe 25 and a cock 24 connected to the lower part of the burette tube 21. The burette part 1 is fixed by the clamp 3. The flat plate-shaped measuring table 13 has a through hole 13a having a diameter of 2 mm and formed in a central part thereof, and is supported by the frame 11 having a variable height. The through hole 13a of the measuring table 13 and the cock 22 of the burette part 1 are connected by the conduit 5. The inner diameter of the conduit 5 is 6 mm.

The measurement part 4 includes a cylinder 31 made of plexiglass, a polyamide mesh 32 bonded to one opening part of the cylinder 31, and a weight 33 movable in a vertical direction in the cylinder 31. The cylinder 31 is placed on the measuring table 13 with the polyamide mesh 32 therebetween. The inner diameter of the cylinder 31 is 20 mm. The mesh size of the polyamide mesh 32 is 75 $\mu$m (200 mesh). The weight 33 has a diameter of 19 mm and a mass of 119.6 g, and can apply a load of 4.14 kPa (0.6 psi) to the water-absorbent resin particles 10a uniformly disposed on the polyamide mesh 32 as described later.

First, the cock 22 and the cock 24 of the burette part 1 were closed, and 0.9 mass% physiological saline adjusted to 25°C was put into the burette tube 21 through the opening at the upper portion of the burette tube 21. Next, the opening at the upper portion of the burette tube 21 was sealed with the rubber stopper 23, and then the cock 22 and the cock 24 were opened. The inside of the conduit 5 was filled with a 0.9 mass% saline 50 so that air bubbles did not enter. The height of the measuring table 13 was adjusted so that the height of the water surface of the 0.9 mass% saline reaching the inside of the through hole 13a was the same as the height of the upper surface of the measuring table 13. After the adjustment, the height of the water surface of the 0.9 mass% saline 50 in the burette tube 21 was read by the scale of the burette tube 21, and the position was defined as a 0 point (value read at 0 second).

In the measurement part 4, 0.10 g of water-absorbent resin particles 10a were uniformly disposed on the polyamide mesh 32 in the cylinder 31, the weight 33 was disposed on the water-absorbent resin particles 10a, and the cylinder 31 was installed so that the central part thereof coincided with a conduit port at the central part of the measuring table 13. The decrease amount (that is, the amount of physiological saline absorbed by the water-absorbent resin particles 10a) Wc (mL) of the physiological saline in the burette tube 21, 60 minutes after the water-absorbent resin particles 10a started to absorb the physiological saline from the conduit 5 was read, and the absorption power of physiological saline under a load of 4.14 kPa of the water-absorbent resin particles 10a was calculated by the following formula.

Absorption power of physiological saline (mL/g) under load of 4.14 kPa = Wc (mL)/mass (g) of water-absorbent resin particles

<Median Particle Diameter (Particle Size Distribution)>

10 g of water-absorbent resin particles were sieved using a sonic vibration type sieving measuring instrument (Robot shifter RPS-01, manufactured by Seishin Enterprise Co., Ltd.), sieves with JIS standard mesh sizes of 850 μm, 600 μm, 500 μm, 425 μm, 300 μm, 250 μm, and 180 μm, and a receptacle. The mass of the particles remaining on each sieve was calculated as a mass percentage with respect to the total

amount. The mass percentage of the particles remaining on each sieve was integrated in a descending order of particle size, and the relationship between the mesh size of the sieve and the integrated value of the mass percentage of particles remaining on the sieve was plotted on a logarithmic probability paper. By connecting the plots on the probability paper with straight lines, a particle diameter corresponding to an integrated mass percentage of 50 mass% was determined, and defined as a median particle diameter.

<Water Content>

2.0 g of the water-absorbent resin particles are placed in an aluminum foil case (No. 8) having a constant weight (W1 (g)) in advance, the mouth of the aluminum foil case is lightly closed, and the total mass W2 (g) of the aluminum foil case containing the sample is precisely weighed. The aluminum foil case containing the sample described above is dried for 2 hours in a hot air dryer (manufactured by ADVANTEC, model: FV-320) set at an internal temperature of 105°C. The dried aluminum foil case containing the sample is allowed to cool to room temperature in a desiccator. The total mass W3 (g) of the aluminum foil case containing the sample after cooling is measured. The water content of the sample is calculated from the following formula.

$$\text{Water content [mass\%]} = [\{(W2-W1)-(W3-W1)\}/(W2-W1)] \times 100$$

[Evaluation of Water-Absorbing Resin Composition]

<Physiological Saline Retention Amount>

The physiological saline retention amount of the water-absorbing resin composition was measured in the same manner as the physiological saline retention amount measured for the water-absorbent resin particles.

<Physiological Saline Absorption Amount Under Load of 4.14 kPa>

The physiological saline absorption amount under a load of 4.14 kPa of the water-absorbing resin composition was measured in the same manner as the physiological saline absorption amount under a load of 4.14 kPa measured for the water-absorbent resin particles.

<Measurement of Water Absorption Rate by Vortex Method>

In a 100 mL beaker, 50±0.1 g of physiological saline adjusted to a temperature of 25±0.2°C in a constant temperature water bath was weighed, and stirred with a magnetic stirrer bar (8 mmφ×30 mm, without a ring) to generate a vortex at a rotation speed of 600 rpm. 2.0±0.002 g of the water-absorbing resin composition was added to the physiological saline at a time, and a time (second) from the addition of the water-absorbing resin composition to a time point at which the vortex in the liquid surface converged was measured, and the time was taken as the absorption rate of the water-absorbing resin composition. This water absorption rate is also expressed as Vortex method or vortex time.

<Median Particle Diameter (Particle Size Distribution)>

The median particle diameter (particle size distribution) of the water-absorbing resin composition was measured in the same manner as the median particle diameter (particle size distribution) measured for the water-absorbent resin particles.

<Water Content>

The water content of the water-absorbing resin composition was measured in the same manner as the water content measured for the water-absorbent resin particles.

<Soluble Component Eluted for 1 Hour after Deterioration Test>

The soluble component eluted for 1 hour after the degradation test was measured by the following procedure.
The "soluble component eluted for 1 hour" in the present invention refers to a water-soluble component measured in a manner such that, in an Ext (water-soluble component) measurement method defined in ERT470.2-02, a 0.90 mass% aqueous sodium chloride solution is changed to an aqueous solution (degradation test solution) obtained by mixing L-ascorbic acid with a 0.90 mass% aqueous sodium chloride solution, in which the water-soluble component is that observed after the water-absorbing resin composition is allowed to stand still in the degradation test solution for 2 hours at 60°C, and then stirred for 1 hour. 200.0 g of an aqueous solution containing 0.05 mass% of L-ascorbic acid and 0.90 mass% of sodium chloride (i.e., a degradation test solution/a mixture of 0.10 g of L-ascorbic acid and 199.90 g of 0.90 mass% aqueous sodium chloride solution) was weighed in a 250 ml plastic container (having an inner lid and an outer lid) into which a rotor having a length of 35 mm had been placed. 1.00 g of the water-absorbing resin composition was added to the aqueous solution, and the plastic container was then sealed with the inner lid and the outer lid. Thereafter, the plastic container was allowed to stand in an incubator adjusted to 60±2°C for 2 hours. After a lapse of 2 hours, the container was taken out from the incubator, and stirred at room temperature for 1 hour using a stirrer (rotation speed: about 500 rpm). The water-soluble component of the water-absorbing resin composition was extracted by the above operations. After the stirring, the extract (which is the aqueous solution) was filtered using one sheet of filter paper (Advantec Toyo Kaisha, Ltd., Product name: JIS P 3801, No. 2, thickness: 0.26 mm, retaining particle diameter: 5 μm), and 50.0 g of the obtained filtrate was weighed and used as a solution for measurement. Next, the solution for measurement was titrated with a 0.1N-NaOH aqueous solution until a pH of 10 was attained, and then titrated with a 0.1N-HCl aqueous solution until a pH of 2.7 was attained. The amounts of the aqueous solutions used in the titration operations were determined as [NaOH] ml and [HCl] ml. The same operation was performed on only 200.0 g of a degradation test solution without adding the water-absorbing resin composition to determine the amounts of the aqueous solutions ([b2NaOH] ml and [b2HCl] ml) used in the blank titration operations. The amount of the aqueous solutions used in titration and the monomer average molecular weight of the water-absorbing resin were used in the following formula to calculate the soluble component eluted for 1 hour.

Soluble component eluted for 1 hour [mass%]=0.1 × monomer average molecular weight × 200 × 100 × ([HCl] - [b2HCl])/(1000 × 1.0 × 50.0)

<Test of Gel Absorbing Artificial Urine>

A gel (swollen gel) in which the water-absorbing resin composition absorbed water was tested using artificial urine. As described in detail below, the evaluation of gel strength after being allowed to stand at 37°C for 14 hours is an index for the evaluation of stability in normal use. The evaluation of the gel strength after being allowed to stand at 37°C for 14 hours and further irradiated with ultraviolet rays is an index for the evaluation of gel separability during a waste treatment.

(Preparation of Artificial Urine)

Artificial urine having the following composition was prepared.

Urea: 20.0 g
Sodium chloride: 8.0 g
Calcium chloride dihydrate: 0.3 g
Magnesium sulfate heptahydrate: 0.8 g
L(+)-ascorbic acid: 0.2 g
Ion exchange water: 970.9 g

(Preparation of Swollen Gel)

39.0 g of the artificial urine was weighed in a beaker having an internal volume of 100 mL (opening inner diameter: 51 mm), and a magnetic stirrer bar (8mmφ×30 mm, no ring) was placed in the beaker. The beaker was disposed on a magnetic stirrer (HS-30D manufactured by iuchi Corporation), and the magnetic stirrer bar was rotated at 600 rpm. Next, 1.00 g of the water-absorbing resin composition was put into the beaker under stirring, and the stirring was performed until a rotating vortex disappeared and a liquid level became horizontal, thereby preparing a swollen gel as a measurement sample. Immediately after preparing the swollen gel, the beaker containing the swollen gel was covered with a wrap (DIARAP manufactured by Mitsubishi Chemical Corporation).

(Measurement of Gel Strength)

The gel strength after being allowed to stand for a predetermined time under each temperature condition was measured using a device having the measurement principle shown in Fig. 2. The device shown in Fig. 2 includes a support unit 50a, a movable base plate 60, a drive unit 70 for driving the movable base plate 60, and a measurement part 80. In the support unit 50a, a frame 53 is fixed to the upper portion of a support pole 52 standing on a support base 51. The movable base plate 60 is attached to the support pole 52 so as to be vertically movable. On the movable base plate 60, a measurement sample (gel) 61 can be mounted. A pulse motor 71 is mounted on the frame 53, and the movable base plate 60 is vertically moved via a wire 73 by rotating a pulley 72. In the measurement part 80, a pressure sensitive shaft 84 is attached to a load cell 81 for measuring distortion caused by deformation via a precision spring 82 and a connecting shaft 83. The pressure sensitive shaft 84 with a disc has a disc at the tip. The diameter of the disc can be changed depending on the measurement conditions. A weight 90 can be mounted on the upper portion of the pressure sensitive shaft 84 with a disc. The operating principle of the device for measuring gel strength is as follows. The precision spring 82 is fixed to the load cell 81 (stress detector) disposed on the precision spring, and the pressure sensitive shaft 84 with a disc disposed below the precision spring is connected to the precision spring. Having a predetermined weight 90 placed thereon, the pressure sensitive shaft 84 with a disc is vertically suspended. The movable base plate 60 on which the measurement sample 61 is placed is elevated at a constant speed by the rotation of the pulse motor 71. A constant speed load is applied to the sample 61 via the spring 82, distortion caused by deformation is measured by the load cell 81, and hardness is calculated by the measurement. The gel strength value (N/m2) was measured using a card meter (Curdmeter-MAX manufactured by Asuka Kiki, product number: ME-500) under the conditions of a disc of the pressure sensitive shaft of 16mmφ, a load of 400 g, a speed of 7 seconds/inch, and a viscous mode.

(Initial Value of Gel Strength)

The swollen gel was allowed to stand under an environment of a temperature of 25±2°C and a relative humidity of 50±10% for 60 minutes after preparation, and then the gel strength (initial value of gel strength) was measured by the method described in the above (Measurement of Gel Strength). The value of the gel strength at this time was defined as gel strength (A).

(Gel Strength after Being Allowed to Stand at 37°C for 14 Hours)

The swollen gel was allowed to stand in a thermo-hygrostat (LHU-113 manufactured by Espec Corporation) set at a temperature of 37±2°C and a relative humidity of 60±10% for 14 hours after preparation, and then the gel strength was measured by the method described in the above (Measurement of Gel Strength). The value of the gel strength at this time was defined as gel strength (B).

(Evaluation of Gel Strength Change Degree and Gel Stability)

From the initial value of the obtained gel strength (gel strength (A)) and gel strength (B), the gel strength change degree (%) was calculated by the following formula. The gel stability was higher as the gel strength change degree was lower, and the stability of the gel was determined from the numerical value of the gel strength change degree using the following criteria.

Gel strength change degree (%) = [(gel strength (A)-gel strength (B))/gel strength (A)] $\times$ 100

<Criteria for Determining Gel Stability>

Gel strength change degree of less than 40%: good
Gel strength change degree of 40% or more: poor

(Gel Strength after Being Allowed to Stand at 37°C for 14 Hours and Further Performing Ultraviolet Irradiation)

The swollen gel was allowed to stand in a thermo-hygrostat (LHU-113 manufactured by Espec Corporation) set at a temperature of 37$\pm$2°C and a relative humidity of 60$\pm$10% for 14 hours after preparation. Thereafter, the swollen gel after being allowed to stand was placed directly under a light source of an ultraviolet irradiation device (SPECTROLINE manufactured by SPECTRONICS, model number: XX-15NF) under an environment of a temperature of 25$\pm$2°C and a relative humidity of 50$\pm$10% while being placed in a beaker (the wrap for covering was removed). At this time, a distance between the surface of the swollen gel and the light source was set to 7 cm. After covering the entire device with a box to shield light, the swollen gel in the beaker was irradiated with ultraviolet rays having a wavelength of 254 nm for 150 minutes. After the ultraviolet irradiation, the gel strength was measured by the method described in the above (Measurement of Gel Strength). The value of the gel strength at this time was defined as gel strength (C).

(Evaluation of Gel Strength Change Degree and Gel Separability after UV Irradiation)

The gel strength change degree (%) after UV irradiation was calculated by the following formula from an initial value of obtained gel strength (gel strength (A)) and a value of the gel strength after ultraviolet irradiation (gel strength (C)). The gel became softer as the gel strength change degree after UV irradiation was larger, and the ease of gel separation operation was determined from the numerical value of the gel strength change degree after UV irradiation using the following criteria.

gel strength change degree (%) after UV irradiation = [(gel strength (A) - gel strength (C))/gel strength (A)] $\times$ 100

<Criteria for Determining Gel Separability>

Gel strength change degree after UV irradiation of less than 40%: poor
Gel strength change degree after UV irradiation of 40% or more: good

[Table 1]

| | Water-absorbent resin particles | | | | | Chelating agent | | | | Water-absorbing resin composition | | | | | | | Gel stability evaluation results | | | Gel separability evaluation results | |
|---|---|---|---|---|---|---|---|---|---|---|---|---|---|---|---|---|---|---|---|---|---|
| | Name | Physiological saline retention amount [g/g] | Physiological saline absorption amount under load of 4.14 kPa [ml/g] | Median particle diameter [μm] | Water content [mass%] | Name | Additive amount [Parts by mass] | Iron (III) chelate stability constant [logKML] | Number of carboxyl groups [Number/molecule] | Name | Physiological saline retention amount [g/g] | Physiological saline absorption amount under load of 4.14 kPa [ml/g] | Water absorption rate [sec] | Median particle diameter [μm] | Water content [mass%] | Soluble component eluted for 1 hour after deterioration test > [mass%] | Initial gel strength (A) [N/m²] | Gel strength after 14 hours (B) [N/m²] | Gel strength change degree [%] | Gel strength after UV irradiation (C) [N/m²] | Gel strength change degree after UV irradiation [%] |
| Example 1 | Water-absorbent resin particles (1) | 40 | 15 | 370 | 8 | EDDS·4H | 0.1 | 20.1[2] | 4 | Water-absorbing resin composition (1) | 40 | 15 | 37 | 370 | 8 | 9 | 7051 | 5146 | 27 | 3451 | 51 |
| Example 2 | Water-absorbent resin particles (1) | 40 | 15 | 370 | 8 | EDDS·3Na | 0.1 | 20.1[2] | 4 | Water-absorbing resin composition (2) | 40 | 15 | 37 | 370 | 5 | 12 | 7048 | 5765 | 18 | 3408 | 52 |
| Example 3 | Water-absorbent resin particles (1) | 40 | 15 | 370 | 8 | GEDTA | 0.1 | 20.5[1] | 4 | Water-absorbing resin composition (3) | 40 | 15 | 38 | 370 | 8 | > 20 | 7596 | 5427 | 29 | 0 | 100 |
| Comparative Example 1 | Water-absorbent resin particles (1) | 40 | 15 | 370 | 8 | DTPA·5Na | 0.1 | 28.6[1] | 5 | Water-absorbing resin composition (4) | 40 | 15 | 36 | 370 | 5 | 10 | 7717 | 7549 | 2 | 6823 | 12 |
| Comparative Example 2 | Water-absorbent resin particles (1) | 40 | 15 | 370 | 8 | EDTMP·2H₂O | 0.1 | 19.6[1] | - | Water-absorbing resin composition (5) | 40 | 15 | 37 | 370 | 7 | > 20 | 7180 | 6344 | 12 | 6338 | 12 |
| Comparative Example 3 | Water-absorbent resin particles (1) | 40 | 15 | 370 | 8 | EDTA-OH | 0.1 | 19.8[1] | 3 | Water-absorbing resin composition (6) | 40 | 15 | 37 | 370 | 8 | > 20 | 7437 | 3615 | 51 | - | - |
| Comparative Example 4 | Water-absorbent resin particles (1) | 40 | 15 | 370 | 8 | MGDA·3Na | 0.1 | 16.5[2] | 3 | Water-absorbing resin composition (7) | 40 | 15 | 37 | 370 | 4 | > 20 | 7100 | 3054 | 57 | - | - |
| Comparative Example 5 | Water-absorbent resin particles (1) | 40 | 15 | 370 | 8 | GLDA·4Na | 0.1 | 15.2[1] | 4 | Water-absorbing resin composition (8) | 40 | 15 | 37 | 370 | 5 | > 20 | 7043 | 2876 | 59 | - | - |
| Comparative Example 6 | Water-absorbent resin particles (1) | 40 | 15 | 370 | 8 | IDS | 0.1 | 13.9[1] | 4 | Water-absorbing resin composition (9) | 40 | 15 | 38 | 370 | 8 | > 20 | 7103 | 2861 | 60 | - | - |
| Comparative Example 7 | Water-absorbent resin particles (1) | 40 | 15 | 370 | 8 | Sodium polyaspartate (Mw 2,000 to 3,000) | 0.1 | 18.5[4] | > 14 | Water-absorbing resin composition (10) | 40 | 15 | 38 | 370 | 5 | > 20 | 7089 | 2867 | 60 | - | - |
| Comparative Example 8 | Water-absorbent resin particles (1) | 40 | 15 | 370 | 8 | HIDS | 0.1 | 15[2] | 4 | Water-absorbing resin composition (11) | 40 | 15 | 36 | 370 | 5 | > 20 | 7032 | 2719 | 61 | - | - |
| Comparative Example 9 | Water-absorbent resin particles (1) | 40 | 15 | 370 | 8 | L(+)-tartaric acid disodium dihydrate | 0.1 | 18.1[1] | 2 | Water-absorbing resin composition (12) | 40 | 15 | 38 | 370 | 8 | > 20 | 7076 | 3097 | 56 | - | - |
| Comparative Example 10 | Water-absorbent resin particles (1) | 40 | 15 | 370 | 8 | Citric acid trisodium dihydrate | 0.1 | 11.9[1] | 3 | Water-absorbing resin composition (13) | 40 | 15 | 38 | 370 | 7 | > 20 | 7104 | 3077 | 57 | - | - |
| Comparative Example 11 | Water-absorbent resin particles (1) | 40 | 15 | 370 | 8 | Pyridine-2,6-dicarboxylic acid | 0.1 | 10.9[1] | 2 | Water-absorbing resin composition (14) | 40 | 15 | 38 | 370 | 7 | > 20 | 7177 | 3841 | 46 | - | - |
| Comparative Example 12 | Water-absorbent resin particles (1) | 40 | 15 | 370 | 8 | EDTA | 0.1 | 25.1[1] | 4 | Water-absorbing resin composition (15) | 40 | 15 | 37 | 370 | 9 | > 20 | 7437 | 3702 | 50 | - | - |
| Comparative Example 13 | Water-absorbent resin particles (1) | 40 | 15 | 370 | 8 | TTHA | 0.1 | 26.8[1] | 6 | Water-absorbing resin composition (16) | 40 | 15 | 36 | 370 | 8 | 12 | 7180 | 6238 | 13 | 6220 | 13 |
| Comparative Example 14 | Water-absorbent resin particles (1) | 40 | 15 | 370 | 8 | Sodium gluconate | 0.1 | 37.2[5] | 1 | Water-absorbing resin composition (17) | 40 | 15 | 38 | 370 | 8 | > 20 | 7119 | 3249 | 54 | - | - |
| Comparative Example 15 | Water-absorbent resin particles (1) | 40 | 15 | 370 | 8 | Sodium glucoheptonate dihydrate | 0.1 | 38.3[5] | 1 | Water-absorbing resin composition (18) | 40 | 15 | 38 | 370 | 7 | > 20 | 7051 | 3374 | 52 | - | - |
| Example 4 | Water-absorbent resin particles (1) | 40 | 15 | 370 | 8 | EDDS·3Na | 0.35 | 20.1[2] | 4 | Water-absorbing resin composition (19) | 40 | 15 | 37 | 370 | 5 | 4 | 8165 | 7864 | 4 | 3874 | 53 |
| Example 5 | Water-absorbing resin composition (20) | 41 | 11 | 361 | 8 | EDDS·3Na | 0.03 | 20.1[2] | 4 | Water-absorbing resin composition (20) | 41 | 11 | 37 | 361 | 8 | 3 | 8042 | 7145 | 11 | 3479 | 57 |
| Example 6 | Water-absorbing resin composition (21) | 39 | 19 | 372 | 9 | EDDS·3Na | 0.01 | 20.1[2] | 4 | Water-absorbing resin composition (21) | 39 | 19 | 37 | 372 | 9 | 1 | 7533 | 6067 | 19 | 4398 | 42 |
| Comparative Example 16 | Water-absorbent resin particles (1) | 40 | 15 | 370 | 8 | MGDA·3Na | 0.35 | 16.5[2] | 3 | Water-absorbing resin composition (22) | 40 | 15 | 37 | 370 | 5 | > 20 | 8165 | 3487 | 57 | - | - |
| Comparative Example 17 | Water-absorbent resin particles (1) | 40 | 15 | 370 | 8 | MGDA·3Na | 0.85 | 16.5[2] | 3 | Water-absorbing resin composition (23) | 40 | 15 | 37 | 370 | 5 | > 20 | 8165 | 1400 | 83 | - | - |
| Example 7 | Water-absorbing resin composition (24) | 35 | 28 | 353 | 7 | EDDS·3Na | 0.03 | 20.1[2] | 4 | Water-absorbing resin composition (24) | 35 | 28 | 39 | 353 | 7 | 4 | 11940 | 10298 | 14 | 6542 | 45 |
| Example 8 | Water-absorbing resin composition (25) | 46 | 10 | 351 | 8 | EDDS·3Na | 0.03 | 20.1[2] | 4 | Water-absorbing resin composition (25) | 46 | 10 | 33 | 351 | 8 | 4 | 7615 | 6697 | 12 | 3475 | 54 |

Notes to Table 1: 1) Dojindo Laboratories, 2018, Product Catalogue, 31th edition, 268-269., 2) Isabel S. S. Pinto, Isabel F. F. Neto, Helena MVM Soares. 2014. Biodegradable chelating agents for industrial, domestic, and agricultural applications–a review. Environ Sci Pollut Res. 21. 11893-11906., 3) Takashi Minoura. 1984. Chelating agent industry. Organic Synthetic Chemistry. 42 (2). 165-169., 4) LANXESS. 2005. BAYPURE GENERAL PRODUCTIMFORMATION. 10. 19., 5) R. M. Smith, A. E. Martell. 1995. Critical Stability Constants. 3rd Edition. Plenum Press.

In the water-absorbing resin compositions of Examples 1 to 8, in addition to the surface-crosslinked water-absorbent resin particles, those having an iron (III) chelate stability constant of 16 logKML or more and 24 logKML or less and 4 or more and 10 or less carboxyl groups are used as a chelating agent. The water-absorbing resin compositions of Examples 1 to 8 had both gel stability of the water-absorbing resin composition when the hygienic material is usually worn and high handleability when the water-absorbing resin composition is subjected to a separation treatment after being irradiated with ultraviolet rays as a waste treatment means. In particular, in the water-absorbing resin compositions of Examples 1 to 2, 4 to 8, the gel strength after UV irradiation was moderate, and the handleability at the time of the separation treatment was particularly excellent.

Reference Signs List

1 Burette part
3 Clamp
4 Measurement part
5 Conduit
10a Water-absorbent resin particles
11 Frame

13 Measuring table
13a Through hole
15 Nylon mesh sheet
21 Burette tube
22 Cock
23 Rubber plug
24 Cock
25 Air introduction pipe
31 Cylinder
32 Polyamide mesh
33 Weight

**Claims**

1. A water-absorbing resin composition comprising water-absorbent resin particles and a chelating agent, wherein

   the water-absorbent resin particles are surface-crosslinked, and
   the chelating agent has an iron (III) chelate stability constant of 16 logKML or more and 24 logKML or less, and 4 or more and 10 or less carboxyl groups.

2. The water-absorbing resin composition according to claim 1, having a gel strength change degree after UV irradiation of 40% or more.

   (Gel strength change degree after UV irradiation)
   According to procedures 1 and 2 below, the gel strength change degree (%) after UV irradiation is calculated by a formula below from an initial value of obtained gel strength (gel strength (A)) and a value of the gel strength after ultraviolet irradiation (gel strength (C)).

   gel strength change degree (%) after UV irradiation = [(gel strength (A) - gel strength (C))/gel strength (A)] × 100

   (Initial value of gel strength (gel strength (A))
   A swollen gel prepared by the following procedure 1 is allowed to stand under an environment of a temperature of $25\pm2$°C and a relative humidity of $50\pm10\%$ for 60 minutes after preparation, and then the gel strength (initial value of gel strength) is measured by the following procedure 2. The value of the gel strength at this time is defined as gel strength (A).
   (Value of gel strength after ultraviolet irradiation (gel strength (C))
   The swollen gel prepared by the following procedure 1 is allowed to stand under an environment of a temperature of $37\pm2$°C and a relative humidity of $60\pm10\%$ for 14 hours after preparation. Thereafter, the swollen gel after being allowed to stand is placed directly under a light source of an ultraviolet irradiation device under an environment of a temperature of $25\pm2$°C and a relative humidity of $50\pm10\%$. At this time, a distance between a surface of the swollen gel and the light source is set to 7 cm. After covering the entire device with a box to shield light, the swollen gel in a beaker is irradiated with ultraviolet rays having a wavelength of 254 nm for 150 minutes. After the ultraviolet irradiation, the gel strength is measured by the following procedure 2. A value of the gel strength at this time is defined as gel strength (C).
   Procedure 1 (preparation of swollen gel)
   In a beaker having an internal volume of 100 mL, 39.0 g of artificial urine is weighed, magnetic stirrer bars are put in the beaker, and the beaker is placed on a magnetic stirrer to rotate the magnetic stirrer bars at 600 rpm. Next, 1.00 g of the water-absorbing resin composition is put into the beaker under stirring, and the stirring is performed until a rotating vortex disappears and a liquid level becomes horizontal, thereby preparing a swollen gel as a measurement sample. Immediately after preparing the swollen gel, the beaker containing the swollen gel is covered with a wrap.
   Procedure 2 (measurement of gel strength)
   A gel strength value (N/m$^2$) is measured using a card meter under conditions of a temperature and standing time of the initial value of the gel strength (gel strength (A)) and the value of the gel strength after ultraviolet irradiation (gel strength (C)).

3. The water-absorbing resin composition according to claim 1 or 2, wherein a content of the chelating agent is 0.001 parts by mass or more and 2 parts by mass or less with respect to 100 parts by mass of the water-absorbent resin particles.

4. The water-absorbing resin composition according to claim 1 or 2, wherein the water-absorbing resin composition has a physiological saline retention amount of 25 g/g or more and 60 g/g or less.

5. The water-absorbing resin composition according to claim 1 or 2, wherein the water-absorbing resin composition has a physiological saline absorption amount of 5 ml/g or more and 40 ml/g or less under a load of 4.14 kPa.

6. An absorber comprising the water-absorbing resin composition according to claim 1 or 2.

7. An absorbent article comprising the absorber according to claim 6.

8. A method for producing the water-absorbing resin composition according to claim 1 or 2, the method comprising:

mixing the water-absorbent resin particles before surface-crosslinking with the chelating agent; and/or
mixing the water-absorbent resin particles after surface-crosslinking with the chelating agent.

FIG. 1

FIG. 2

| | **INTERNATIONAL SEARCH REPORT** | International application No. |
|---|---|---|
| | | **PCT/JP2023/012459** |

**A.    CLASSIFICATION OF SUBJECT MATTER**

*B01J 20/26*(2006.01)i; *A61F 13/15*(2006.01)i; *A61F 13/505*(2006.01)i; *A61F 13/53*(2006.01)i; *B01J 20/30*(2006.01)i
FI:    B01J20/26 D; A61F13/15 140; A61F13/505; A61F13/53 300; B01J20/30

According to International Patent Classification (IPC) or to both national classification and IPC

**B.    FIELDS SEARCHED**

Minimum documentation searched (classification system followed by classification symbols)

B01J20/00-20/34, A61F13/15-13/84

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | WO 2018/062539 A1 (NIPPON SHOKUBAI CO., LTD.) 05 April 2018 (2018-04-05) claims, paragraphs [0031]-[0435] | 1-8 |
| A | WO 2021/162082 A1 (NIPPON SHOKUBAI CO., LTD.) 19 August 2021 (2021-08-19) | 1-8 |
| A | CN 105311669 A (JIANGSU QIANZE HEALTH TECHNOLOGY CO., LTD.) 10 February 2016 (2016-02-10) | 1-8 |

☐ Further documents are listed in the continuation of Box C.        ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 June 2023** | **20 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/012459**

| Patent document cited in search report | | | Publication date (day/month/year) | Patent family member(s) | | | Publication date (day/month/year) |
|---|---|---|---|---|---|---|---|
| WO | 2018/062539 | A1 | 05 April 2018 | US | 2019/0338082 | A1 | |
| | | | | claims, paragraphs [0044]-[0520] | | | |
| | | | | CN | 110023412 | A | |
| | | | | DE | 17856486 | T1 | |
| | | | | EP | 3521376 | A1 | |
| | | | | KR | 10-2019-0077359 | A | |
| WO | 2021/162082 | A1 | 19 August 2021 | EP | 4104942 | A1 | |
| CN | 105311669 | A | 10 February 2016 | (Family: none) | | | |

Form PCT/ISA/210 (patent family annex) (January 2015)

27

**EP 4 501 447 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005029751 A